# NEUE EUROPÄISCHE PATENTSCHRIFT

(11) **EP 1 976 524 B2**
(45) Veröffentlichungstag und Bekanntmachung des Hinweises auf die Entscheidung über den Einspruch: **10.08.2016**
(45) Hinweis auf die Patenterteilung: 28.04.2010
(21) Anmeldenummer: 07704010.3
(22) Anmeldetag: 19.01.2007
(51) Int. Cl.: A61K 31/485, A61K 45/06, A61P 25/36, A61P 25/04

(54) **VERWENDUNG EINER KOMBINATION VON MORPHIN UND MINDESTENS EINEM OPIATANTAGONISTEN ZUR BEHANDLUNG VON OPIATABHÄNGIGKEIT UND ZUR VERHINDERUNG DES NICHT-ORALEN OPIATMISSBRAUCHS BEI OPIATSÜCHTIGEN**
USE OF A COMBINATION OF MORPHINE AND AT LEAST ONE OPIATE ANTAGONIST FOR TREATMENT OF OPIATE DEPENDENCY AND FOR PREVENTION OF NON-ORAL OPIATE ABUSE IN OPIATE ADDICTS
UTILISATION D'UN MELANGE DE MORPHINE ET D'AU MOINS UN ANTAGONISTE DES OPIACES POUR LE TRAITEMENT DE LA DEPENDANCE AUX OPIACES ET POUR EVITER L'ABUS NON ORAL D'OPIACES CHEZ LES PERSONNES DEPENDANTES AUX OPIACES

(30) Priorität: 19.01.2006 EP 06100578
(43) Veröffentlichungstag der Anmeldung: 08.10.2008
(73) Patentinhaber: EURO-CELTIQUE S.A., 2350 Luxembourg (LU)
(72) Erfinder: HERMANN, Lars, CH-8834 Schindellegi (CH)
(74) Vertreter: Ledl, Andreas
(86) Internationale Anmeldenummer: PCT/EP2007/050540
(87) Internationale Veröffentlichungsnummer: WO 2007/082935

(56) Entgegenhaltungen:
- WO-A-01/58447
- WO-A-97/33566
- WO-A-99/32119
- WO-A1-01/58451
- WO-A1-02/092060
- DE-A1- 4 325 465
- GB-A- 1 390 772
- US-A- 4 769 372
- US-A- 5 580 876
- US-A1- 2005 063 909
- MITCHELL T.B. ET AL: 'Comparative pharmacodynamics and pharmacokinetics of methadone and slow-release oral morphine for maintenance treatment of opioid dependence' DRUG AND ALCOHOL DEPENDENCE Bd. 72, 2003, Seiten 85 - 94
- LOIMER N. ET AL: 'Combined Naloxone/Methadone Preparations for Opiate Substitution Therapy' JOURNAL OF SUBSTANCE ABUSE TREATMENT Bd. 8, 1991, Seiten 157 - 160
- KRAIGHER D. ET AL: 'Use of Slow-Release Oral Morphine for the Treatment of Opioid Dependence' EUROPEAN ADDICTION RESEARCH Bd. 11, 2005, Seiten 145 - 151
- F. VON BRUCHHAUSEN ET AL.: 'Hagers Handbuch der Pharmazeutischen Praxis', Bd. 9, 1995, SPRINGER-VERLAG, BERLIN Seite 34
- 'Goodman & Gilman's The Pharmacological Basis of Therapeutics', Bd. 10, MCGRAW-HILL, NEW YORK Seite 1986
- Fachinformation zu Substitol, S4, Pkt 6.9

## Beschreibung

Die vorliegende Erfindung betrifft die Verwendung einer Kombination aus Morphin und mindestens einem Opiatantagonisten zur Behandlung von Opiatabhängigkeit, beim Menschen, wobei der Opiatantagonist Naloxon ist.

Die Drogensucht ist nach wie vor ein Problem in unserer Gesellschaft. Es ist hinlänglich bekannt, dass die Einnahme bestimmter Substanzen wie beispielsweise Heroin zu einer Abhängigkeit führt. Man unterscheidet hierbei zwischen einer physischen Abhängigkeit und der psychischen Drogenabhängigkeit (Drogensucht). Die physische Drogenabhängigkeit tritt bei einem abrupten Absetzen oder einer signifikanten Verringerung der Menge der eingenommenen Substanz auf. Es kommt zu Entzugserscheinungen wie Erbrechen und Krämpfen des Magen-Darm-Trakts. Hiervon unterscheidet man die eigentliche Drogensucht, die psychische Abhängigkeit, welche als eigene neurologische Erkrankung angesehen wird. Anzeichen von Drogensucht sind die fehlende Kontrolle über die eigene Verwendung der Droge bis hin zur Selbstgefährdung sowie das zwanghafte Verlangen, in den Besitz der Droge zu gelangen.

Unter Drogensucht im Sinne dervorliegenden Erfindung wird insbesondere eine Opiatabhängigkeit verstanden (psychische und Verhaltensstörungen durch Opioide; F11 der ICD-10). Besonders gut kann mit dem vorliegenden Verfahren eine Heroinabhängigkeit oder Opiatabhängigkeit therapiert werden.

Drogensucht wird derzeit behandelt, indem man den Patienten substituiert, d.h. von einer gefährlichen auf eine weniger gefährliche Droge umstellt oder den Patienten durch Dosisreduktion unter kontrollierten Bedingungen reduziert. Aus verschiedenen Gründen findet hierbei aber nicht jede Droge Akzeptanz bei den Patienten. Als Substitutionsmittel wird Methadon, Heroin, Buprenorphin und Morphin eingesetzt. Methadon ist ein synthetisches Opiat mit der Formel:

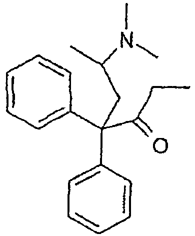

Methadon wird seit den 60er Jahren des 20. Jahrhunderts als Substitutionsmittel eingesetzt und zwar sowohl in der optisch aktiven L-Form als auch in Form des Racemats. Methadon ist geeignet, die Entzugserscheinungen vor allem bei Heroinpatienten gut zu mildem. Es hat eine längere Wirkdauer als Heroin, kann ohne nennenswerten Wirkungsverlust oral eingenommen werden und ruft keinen "Kick" hervor (d.h. das besonders euphorische Gefühl, das durch z.B. Heroin ausgelöst wird und zur Drogensucht führt). Um eine missbräuchliche intravenöse Gabe zu verhindern, wird Methadon in Suchtbekämpfungsprogrammen oft als Sirup, gemischt mit Zucker oder Orangensaft verabreicht.

Allerdings ist die Wirkung von Methadon individuell verschieden und dieses daher nicht einfach zu verabreichen. Methadon macht ebenfalls hochgradig abhängig. Die Entzugserscheinungen bei Methadon sind sogar länger anhaltend als bei Heroin. Methadon führt wie alle Opiate zu Verstopfung, da es lähmend auf die Magen-DarmMuskulatur wirkt. Zudem scheint Methadon erhebliche Nebenwirkungen zu besitzen.

Aus der WO 97/33566 A2 ist eine Dosierform mit retardiertem Morphin und retardiertem Naloxon bekannt. Diese Dosierform enthält eine semipermeable Wand, die einen zweischichtigen Kem aus einem Opioid (äußere Schicht) und - davon getrennt - einem Antagonisten (innere Schicht) aufweist. Durch die physikalische Trennung des Opioids und des Antagonisten ist es für den Drogenabhängigen ein Leichtes, das Opioid durch Trennmaßnahmen zu erhalten, in dem er beispielsweise zunächst die äußere Wand der Dosierform abkratzt und dann die äußere Hülle des freigelegten Kerns abträgt.

Aus der WO 01/58447 A1 sind Präparate mit kontrollierter Freisetzung auf Basis von retardiertem Morphin und retardiertem Antagonisten bekannt. Es findet sich allerdings kein Hinweis dazu, dass der Antagonist eine geringe Bioverfügbarkeit aufweisen soll.

Aus der WO 99/32119 A1 und der DE 43 25 465 A1 sind Präparate zur Schmerztherapie auf Basis von retardiertem Morphin und nicht-retardiertem Naloxon bekannt, ohne den Anwendungsbereich dervorliegenden Erfindung zu berühren.

Es sind aus dem Stand der Technik auch Kombinationspräparate aus bestimmten Opioid-Analgetika und Naloxon bekannt. Das Handelsprodukt Talwin®Nx enthält Pentazocin und Naloxon. Das Produkt Valoron®N enthält Tilidin und Naloxon. Es handelt sich hierbei aber um Schmerzpräparate, bei denen das zugesetzte Naloxon eine missbräuchliche Verwendung als Droge verhindern soll. Es wurde bislang nicht erwogen, diese Handelsprodukte als Drogensubstitutionsmittel einzusetzen. Im Stand der Technik wurde vielmehr versucht, die Verwendung der entsprechenden Präparate als Droge zu verunmöglichen.

Es ist weiterhin bereits eine Kombination von Buprenorphin und Naloxon als Drogensubstitutionsmittel bekannt. Aufgrund der schwachen intrinsischen Aktivität des Stoffes ist die Substanz Buprenorphin nur für einen Teil der Opiatabhängigen als Substitutionsmittel geeignet.

Es besteht daher ein Bedarf nach einer anderen und verbesserten Behandlung von Drogensucht.

Gemäß der vorliegenden Erfindung wurde überraschend gefunden, dass eine Kombination aus Morphin und mindestens einem Opiatantagonisten mit einer Bioverfügbarkeit von weniger als 5% bei oraler Gabe ein geeignetes Substitutionsmittel zur Behandlung der Drogensucht darstellt wobei der Opiatantagonist Naloxon ist. Gleichzeitig wird der nicht-orale Abusus zuverlässig verhindert.

Die vorliegende Erfindung betrifft daher die Verwendung einer nicht-trennbaren Kombination von Morphin und mindestens einem Opiatantagonisten mit einer Bioverfügbarkeit von weniger als 5% bei oraler Gabe zur Herstellung eines nur oral darzureichenden Arzneimittels zur Behandlung von Drogensucht beim Menschen, insbesondere zur Substitutionstherapie bei Drogensüchtigert wobei der Opiatantagonist Naloxon ist.

Die erfindungsgemäße Substanzkombination stellt überraschenderweise ein ideales Substitutionsmittel dar. Es hat sich im Laufe von Untersuchungen gezeigt, dass ihr Rezeptorprofil und ihr physiologischerAbbauweg im Rahmen einer Substitutionstherapie besonders vorteilhaft sind.

### Abusus:

Durch die erfindungsgemäßen Substanzkombinationen wird deren nicht-oraler Abusus verhindert.

Unter nicht-oralem Abusus ist der intravenöse, pulmonale (durch Rauchen), nasale, sublinguale oder rektale Abusus zu verstehen. Hierbei versucht der Drogensüchtige die berauschende Wirkung durch vom Hersteller eines entsprechenden Präparats nicht vorgesehene Applikationswege zu erhalten. Beispielsweise wird ein Präparat geschmolzen oder aufgelöst und intravenös injiziert oder in Präparat auf Alufolie verbrannt und der Rauch inhaliert wird (pulmonaler Missbrauch; Folienrauchen).

Durch die erfindungsgemäße Auswahl von Antagonisten mit geringer Bioverfügbarkeit bei oraler Applikation, also einer Bioverfügbarkeit von unter 5% bei oraler Gabe, und eines Agonisten mit für physiologische Blutspiegel ausreichende Bioverfügbarkeit bei oraler Gabe, wird sichergestellt, dass nur die orale Einnahme des erfindungsgemäßen Kombinationspräparats zu einem guten Substitutionsergebnis führt. Andere Arten der Einnahme, beispielsweise intravenös, pulmonal, nasal oder rektal, führen unmittelbar unter Umgehung des First-Pass-Metabolismus durch die Leber und/oder des Metabolismus in der Darmwand und die spaltende Aktivität der Verdauungsenzyme zu Entzugserscheinungen, da die Wirkung des Antagonisten voll eintritt.

### Nicht-Trennbarkeit:

Viele Drogenabhängige trennen die ihnen im Rahmen einer Substitutionstherapie angebotenen KombinationsPräparate auf, um an den jeweiligen süchtig machenden Inhaltsstoff zu gelangen. Hierzu lösen sie bei mehrschichtigen Präparaten die einzelnen Schichten in Lösungsmittel, beispielsweise Ethanol, auf und erhalten durch Eindampfen der so gewonnen Lösung das reine Suchtmittel. Auch findet eine manuelle Auftrennung, beispielsweise durch Abkratzen oder Aufbrechen der äußeren Hülle eines Präparats und Freilegen der innen gelegenen Suchtmittel statt. Die Inhaltsstoffe von Präparaten, die aus einzelnen erkennbaren Einheiten, beispielsweise einerseits Tabletten mit dem Agonisten und andererseits Tabletten mit dem Antagonisten bestehen, können ohnehin leicht von einander getrennt werden.

Infolgedessen, besteht ein besonderer Vorteil der vorliegenden Erfindung darin, dass der Patient, also der Drogensüchtige, das Morphin oder allgemeiner das Opiat nicht vom Antagonisten trennen kann.

Geeignet sind dazu beispielsweise Darreichungsformen, in denen Agonist und Antagonist als eine Pulver- oder Granulatmischung vorliegen. Geeignete Pulver können Komgrößen von 25 ∼m bis 0,1 mm, geeignete Granulate können Kömgrößen von 0,1 mm bis 2 mm aufweisen Ueweils maximale Größe aller in der Mischung vorliegenden Partikel). Die Bestimmung der Teilchengrößen kann durch Trennverfahren durchgeführt werden, wobei Siebtrennung besonders geeignet ist. Dem Drogensüchtigen ist es bei dieser Ausführungsform aufgrund der Vermischung und der visuellen Ununterscheidbarkeit der verschiedenen Partikel nicht möglich, die einzelnen Partikel der Mischung sicher in Agonisten und Antagonisten aufzutrennen.

Geeignet sind ferner Darreichungsformen, in denen Agonist und Antagonist gemeinsamin einer Matrix eingebettet vorliegen und beispielsweise zu einer Tablette verpresst werden. Auch hier ist es dem Drogensüchtigen nicht mehr möglich, die wirksamen Bestandteile von der Matrix zu trennen. Ein Auflösen in Lösungsmittel führt dazu, dass alle Inhaltsstoffe gleichermaßen aufgelöst werden und eine Trennung mit dem .Drogensüchtigen zur Verfügung stehenden Mitteln nicht mehr möglich ist. Ein mechanisches Zerteilen der die aktiven Inhaltsstoffe enthaltenden Matrix führt ebenfalls nicht dazu, dass der Agonist vom Antagonisten getrennt erhalten werden kann.

Grundsätzlich wird in der vorliegenden Erfindung eine nicht-trennbare Darreichungsform von Agonisten und Antagonisten gewählt, die ein Auftrennen der Mischung in Agonisten und Antagonisten mit mechanischen und/oder visuellen Trennmethoden, also beispielsweise durch Inaugenscheinnahme und anschließendem manuellen Auftrennen der unterschiedlichen Bestandteile der Mischung, oder durch Auflösen der Mischung und anschließendem Abdampfen und/oder Ausfällen der einzelnen Bestandteile der Mischung nicht ermöglicht.

Vermieden wird also eine leichte Trenn- oder Ablösbarkeit mit physikalischen und/oder chemischen Verfahren, insbesondere einfachen Haushaltstrennungsverfahren wie Filtrieren durch Kaffeefilter, Lösen in leicht zugänglichen Lösungsmitteln, Filtrieren durch Zigarettenfilter, Abseien, Trennung aufgrund unterschiedlichem Molekulargewicht und/ oder unterschiedlicher Schwimmeigenschaften (Abschöpfen).

Im Rahmen der erfindungsgemäßen Verwendung in der Substitutionstherapie wird Morphin verwendet.

Morphin ist ein Opiat mit der Formel:

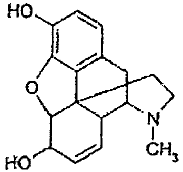

Morphin, umgangssprachlich auch als Morphium bezeichnet, ist ein sehr wirksames Schmerzmittel, das aus den Kapseln des Schlafmohns gewonnen werden kann. Es macht ebenso wie Heroin süchtig, ist aber weniger wirksam.

Gemäß der vorliegenden Erfindung wird das Morphin in retardierter oder nicht-retardierter Form verabreicht.

Unter einer retardierten Form (oder Retardform) eines Arzneimittels versteht man eine Darreichungsform, bei der der Wirkstoff verlangsamt freigesetzt wird. Mit Hilfe von retardierten Formen kann man den Wirkstoff in kontrollierter Weise im Körper freisetzen und somit möglicherweise gefährliche Konzentrationsspitzen des Wirkstoffs im Blut verhindern. Durch die kontrollierte Abgabe des Wirkstoffs wird eine verlängerte Wirkdauer des Arzneimittels im Patienten erzielt. Der Wirkstoff muss daher weniger oft eingenommen werden.

Gemäß der vorliegenden Erfindung ist eine retardierte Form von Morphin bevorzugt.

Diese kann eine nur einmal oder zweimal tägliche Einnahme des Opiats ermöglichen.

Im Sinn der vorliegenden Efindung werden die Begriffe "retardierte Form", "Retardform" und" sustained release-Fom" synonym verwendet.

Durch Verwendung von Morphin in retardierter Form muss das Substitutionsmittel weniger häufig eingenommen werden. Aufgrund der länger anhaltenden Wirkung des Morphins wird beim Drogensüchtigen das Verlangen gemildert, sich baldmöglichst neues Rauschmittel besorgen zu müssen. Zudem werden durch die Retardform die Gefahren einer Überdosis gemildert, da der Wirkstoff in kontrollierter Weise unter Vermeidung von Konzentrationsspitzen ins Blut gegeben wird.

Retardierte Formen von Opiaten und insbesondere von Morphin sind dem Fachmann hinlänglich bekannt. Gemäß der vorliegenden Erfindung kann jede retardierte Form von Morphin eingesetzt werden. Bevorzugt ist gemäß der vorliegenden Erfindung eine Formulierung, bei der das Morphin auf einem Polymer, beispielsweise einem hydrophilen Polymer, adsorbiert und in einer Matrix, beispielsweise einer hydrophoben Matrix, eingebettet ist. DerAntagonist befindet sich vorzugsweise ebenfalls in der Matrix, um die erfindungsgemäße Nicht-Trennbarkeit sicher zu stellen. Vorzugsweise handelt es sich bei dem Polymer um ein Cellulose-Polymer und bei der Matrix um ein Wachs. Bei Kontakt mit dem Magensaft bildet das Polymer ein Gel, durch welches der Wirkstoff nur unter Verzögerung hindurch treten kann. Eine andere bevorzugte Retard-Formulierung ist dadurch gekennzeichnet, dass in einer flüssigen Darreichungsform das Morphin in einem Ethylcellulose-Polymer suspendiert und nur verzögert freigesetzt wird. Eine weitere gebräuchliche Retardform ist eine mit einer Schutzschicht überzogene Tablette. Die Schutzschicht löst sich im Magensaft nur langsam auf, und der Wirkstoff wird entsprechend verzögert freigesetzt.

Das Morphin kann gemäßdervorliegenden Erfindung in allen üblichen Darreichungsformen verabreicht werden. Bevorzugt sind physiologisch akzeptable Salze wie Hydrochloride, Hydrate, Sulfate, Chlorate und quartäre Salze. Besonders bevorzugte Salze des Morphins sind Morphinhydrochlorid, Morphinsulfatpentahydrat, Morphinchlorat, Morphinmethobromid und andere quartäre Salze des Morphins sowie Mörphin-N-oxid.

Die Gefahr einer missbräuchlichen intravenösen Einnahme des erfindungsgemäßen Präparats kann auch weiter dadurch vermindert werden, dass das Morphin in retardierter Form vorliegt.

In dem erfindungsgemäßen Präparat sind 1 bis 2000 mg, vorzugsweise 100 mg bis 1500 mg Morphin enthalten. Die Menge kann entsprechend den Bedürfnissen des Patienten angepasst werden.

### Antagonist:

Gemä er vorliegenden Erfindung wird das Morphin in Verbindung mit mindestens einem Antagonisten gegeben. Der Opiatantagonist ist Naloxon oder eines seiner Derivate oder Salze. Naloxon weist folgende Strukturformel auf:

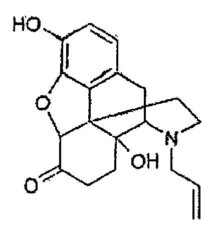

Das Naloxon, kann gemäß der vorliegenden Erfindung in allen üblichen Darreichungsformen verabreicht werden. Bevorzugt sind physiologisch akzeptable wasserlösliche Salze wie Hydrochlorid oder Hydrochloriddihydrat. Das Naloxon liegt im erfindungsgemäßen Präparat entweder in retardierter oder in nicht-retardierter Form vor. Bevorzugt ist die nicht-retardierte Form.
Naloxon wirkt als Opiatantagonist genau entgegengesetzt zu Opiatagonisten wie Morphin:

Naloxon blockiert die Opiatrezeptoren und vermindert die Wirkung des Morphins. Bei entsprechend hoher Dosierung schaltet Naloxon die Wirkung von Morphin vollständig aus, und es kommt zu Entzugserscheinungen. Dieser Effekt ist bekannt und wird beispielsweise zur Behandlung von Opiat-Vergiftungen durch Gabe von Naloxon ausgenützt.

Bemerkenswerterweise tritt die antagonistische Wirkung von Naloxon bei intravenöser Gabe in signifikantem Umfang auf, bei oraler Gabe jedoch kaum. Der Grund hierfür liegt darin, dass Naloxon bei oraler Darreichung einem ausgeprägten First-Pass-Metabolismus unterliegt und daher rasch abgebaut wird. Mit anderen Worten ist die Bioverfügbarkeit von oral verabreichtem Naloxon sehr gering, mitunter geringer als 5%, und die opioide Wirkung des Morphins wird durch das Naloxon kaum beeinträchtigt. Bei intravenöser oder anderen missbräuchlicher Einnahme würde das Naloxon jedoch die opioide Wirkung des Morphins ertieblich beeinträchtigen. Mit anderen Worten ist der Patient beim erfindungsgemäßen Präparat dazu genötigt, das Mittel oral einzunehmen. Die intravenöse, nasale oder pulmonale Einnahme ist bei Drogensüchtigen aufgrund des schnellen Wirkungseintritts ("Kick") zweifellos die Darreichungsroute der Wahl. Mit dem erfindungsgemäßen Präparat würde die intravenöse Einnahme aber nur zur Frustration führen, da die Wirkung des Morphins durch das intravenöse wirksame Naloxon erheblich reduziert, im Extremfall sogar unterbunden würde, was zum unmittelbaren Eintreten von Entzugserscheinungen führen würde.

Die im Rahmen der vorliegenden Erfindung verwendbaren Antagonisten weisen eine Bioverfügbarkeit von weniger als 5%, bevorzugt weniger als 3%, besonders bevorzugt weniger als 1 % auf. Unter Bioverfügbarkeit ist hierbei der Anteil in Prozent des Wirkstoffes, der bei oraler Verabreichung der erfindungsgemäßen Mischung unverändert im Blut erscheint, zu verstehen. (Die Bioverfügbarkeit eines intravenös injizierten Arzneimittels ist definitionsgemäß 100% Zur weitergehenden Definition wird auf Rainer K. Liedtke, Wörterbuch der Klinischen Pharmakologie, Gustav Fischer Verlag, Stuttgart, New York, 1980 verwiesen. Die Bioverfügbarkeit ist auch im WHO Annex 9, 1996 definiert.) Durch die geringe Bioverfügbarkeit wird sicher gestellt, dass der Drogenabhängige beim Einnehmen des erfindungsgemäßen Präparats keinen "Kick" verspürt, sondern ein relativ geringer Level an Antagonisten im Blut des Patienten erhalten wird. Überraschend ist in diesem Zusammenhang, dass trotz der geringen Bioverfügbarkeit eine Substitutionstherapie mit den weiter unten angeführten Vorteilen ermöglicht wird.

Efindungsgemäßwird mindestens ein Antagonist verwendet. Bevorzugt ist die Verwendung eines, zweier oder dreier Antagonisten in Kombination mit Morphin. Besonders bevorzugt ist die Kombination von Morphin und Naloxon.

Die möglichen Kombinationen retardierter und nicht-retardierter Wirkstoffe umfassen retardiertes Morphin und nicht-retardierten Antagonisten, retardiertes Morphin und retardierten Antagonisten, nicht-retardiertes Morphin und nicht-retardierten Antagonisten sowie nicht-retardiertes Morphin und retardierten Antagonisten. Naloxon ist jeweils der Antagonist.

Erfindungsgemäß ist die Menge an Naloxon in dem Präparat so hoch, dass die durch das Morphin verursachten unerwünschten Nebenwirkungen, beispielsweise Verstopfung, verringert werden.

Es hat sich zudem gezeigt, dass durch die Anwesenheit von Naloxon die sonst auftretende Toleranz des Patienten gegen Morphin verringert wird.

Somit weist das erfindungsgemäße Präparat folgendes überraschendes Profil auf:
- Verringerung oder Vermeidung der morphinverursachten Verstopfung;
- Verringerung oder Vermeidung der Toleranzentwicklung gegen Morphin;
- Verhinderung einer missbräuchlichen intravenösen, inhalativen oder nasalen Einnahme (abusus);
- Hervorragende Eignung als Substitutionsmittel;
- Verringerung oder Vermeidung des durch die morphinverursachte Verstopfung ausgelösten üblen Geruchs, den substituierte Patienten abgeben;
- Verringerung oder Vermeidung der durch Substitutionsmittel ausgelösten sexuellen Dysfunktion.

Überdies kann die erfindungsgemäße Verwendung zu einer Verringerung oder Vermeidung der Veränderung des Body Mass Indexes (BMI) sowie einer Verminderung oder Vermeidung der Zahnzerstörung, die bei substituierten Patienten zu beobachten ist, beitragen.

Pro 100 mg retardiertem Morphin sind erfindungsgemäß 0,1 bis 10 mg Naloxon, vorzugsweise 1 bis 5 mg Naloxon oder 1 bis 5 mg pro Tablette enthalten.

Eine besonders bevorzugte Ausführungsform der vorliegenden Erfindung ist ein Präparat mit 100 mg bis 1500 mg retardiertem Morphin in Kombination mit 1 mg bis 5 mg Naloxon. Obwohl gemäß der vorliegenden Erfindung vorzugsweise Naloxon als zusätzlicher Wirkstoff eingesetzt wird, sind prinzipiell jedoch alle Opiatantagonisten mit entsprechender geringer Bioverfügbarkeit bei oraler Gabe einsetzbar. Derartige Verbindungen sind dem Fachmann bekannt. Hinsichtlich Mengenangaben und Darreichungsart gilt für diese Substanzen analog das vorstehend zu Naloxon Ausgeführte.

Das erfindungsgemäße Präparat ist dadurch gekennzeichnet, dass es aus den vorstehenden Gründen nicht intravenös eingenommen werden kann. Das erfindungsgemäße Präparat kann oral (z.8. als Lösung oder Tablette) eingenommen werden. Dem Fachmann sind die entsprechenden Darreichungsformen bekannt. Das erfindungsgemäße Präparat enthält je nach spezifischer Darreichungsform die hierfür üblichen Zusatzstoffe, welche dem Fachmann ebenfalls hinreichend bekannt sind und hier nicht weiter erläutert werden müssen.

Die Erfindung wird durch die nachfolgenden Beispiele näher erläutert, ohne dass sie durch diese beschränkt werden soll.

### Beispiele

Das in den folgenden Beispiele verwendete Morphin ist ein Pulver mit einer Korngröße < 400 µm; Hersteller: McFarland. Das verwendete Naloxon stammt von Sanofi-Aventis. Das Eudragit von Röhm GmbH, Darmstadt, Deutschland.

### Beispiel 1: Morphin/Naloxon-Retardkapsel

Im Folgenden wird die Herstellung von Naloxon-Retardpellets und Morphin-Retardpellets beschrieben, welche in eine Hartgelatinekapsel gefüllt werden.

### Beispiel-1A: Naloxon-HCl-Retardpellets

**Formulierung:**

| Inhaltsstoff | Menge/Einheit | Menge/Charge (g) |
|---|---|---|
| Naloxon HCl | 2,0 | 33,3 |
| Eudragit RSPO | 70,0 | 1166,7 |
| Eudragit RLPO | 8,0 | 133,3 |
| Stearinsäure | 40,0 | 666,7 |
| Gesamt | 120,0 | 2000,0 |

### Verfahren:

Naloxon HCl, Eudragit RSPO, Eudragit RLPO und Stearinsäure werden in einem Zwillingstrommelmischer vermischt. Das vermischte Material wird fortlaufend in einen Doppelschneckenextruder gegeben und die resultierenden Stränge auf einem Förderband gesammelt. Die Stränge werden auf dem Förderband gekühlt. Die gekühlten Stränge werden in einem Pelletierer zu Pellets geschnitten. Die Pellets werden ausgesiebt und der gewünschte Siebanteil gesammelt.

### Beispiel 1B: Morphin-HCl-Retardpellets

**Formulierung:**

| Inhaltsstoff | Menge/Einheit | Menge/Charge (kg) |
|---|---|---|
| Morphin HCl | 12,0 | 3,2 |
| Eudragit RSPO | 76,5 | 20,4 |
| Ethylcellulose | 4,5 | 1,2 |
| Stearylalkohol | 27,0 | 7,2 |
| Gesamt | 120,0 | 32,0 |

### Verfahren:

Stearylalkohol- Flocken werden durch eine Schlagmühle geführt. Morphin HCl, Eudragit RSPO, Ethylcellulose und Stearylalkohol werden in einem Zwillingstrommelmischer vermischt. Das vermischte Material wird fortlaufend in einen Doppelschneckenextruder gegeben und die resultierenden Stränge auf einem Förderband gesammelt. Die Stränge werden auf dem Förderband gekühlt. Die gekühlten Stränge werden mit einem Pelletierer zu Pellets geschnitten. Die Pellets werden ausgesiebt und der gewünschte Siebanteil gesammelt.

Die in den Beispielen 1A und 1 B hergestellten Pellets werden im Verhältnis 1:1 in eine Hartgelatinekapsel gefüllt und diese verschlossen.

### Beispiel 2: Oplat-Agonist/Antagonist-Retardgranulat (als Tablette gepresst)

Im Folgenden wird die Herstellung von Retardtabletten, welche Morphin HCl und Naloxon HCl enthalten, beschrieben, wobei beide Wirkstoffe als Granulat vorfiegen. Die das Morphin und das Naloxon enthaltenden Granulate werden in einer Matrix mit kontrollierter Freisetzung dispergiert. Die Granulate werden mit geschmolzenem Wachs (Stearylalkohol) kombiniert, um gewachste Granulate zu erhalten, welche dann gemahlen, mit anderen Hilfsstoffen vermischt und zu Tabletten gepresst werden.

**Formulierung:**

| Inhaltsstoff | Menge/Einheit (mg) | Menge/Charge (g) |
|---|---|---|
| Morphin HCl | 10,0 | 11,00 |
| Naloxon HCl | 0,50 | 0,55 |
| Sprühgetrocknete lactose | 68,75 | 75,62 |
| Povidon | 5,00 | 5,50 |
| Eudragit RS 30D (Trockengew.) | 10,00 | 11,00 |
| Triacetin | 2,00 | 2,20 |
| Stearylalkohol | 25,00 | 27,50 |
| Magnesiumstearat | 1,25 | 1,38 |
| Opadry-Weiß | 5,00 | 5,50 |
| Gereinigtes Wasser | | 31,16* |
| Gesamt | 127,5 | 140,25 |
| * Verbleibt lediglich als Restfeuchte im Produkt. | | |

### Verfahren:

Mahlen: Eudragit wird mit Triacetin durch Mischen aufgeweicht und Naloxon Hel in dieser lösung gelöst.
Granulierung: Morphin Hel, sprühgetrocknete lactose und Povidon wird in einen Wirbelbettgranulierer gegeben und die im vorhergehenden Schritt hergestellte lösung zugegeben.
Mahlen: Das Granulat wird durch eine rotierende Gebläsemühle geführt.
Trocknen: Falls der Feuchtegehalt des Granulats zu hoch ist, wird getrocknet.
Wachsen: Das Granulat wird durch Zugabe von geschmolzenem Stearylalkohol während des Mischens gewachst.
Kühlen: Das gewachste Granulat wird in einem Wirbelbetttrockner gekühlt.
Mahlen: Das gekühlte und gewachste Granulat wird durch eine rotierende Gebläsemühle geführt. Vermischen: Das gemahlene und gewachste Granulat wird mit Magnesiumstearat vermischt.
Verpressen: Das resultierende Granulat wird unter Verwendung einer Tablettenpresse verpresst.
Beschichtung: Das Opadry-Weiß wird in gereinigtem Wasser dispergiert, um eine Beschichtungslösung zu erhalten, welche auf den Tablettenkern gegeben wird.

### Beispiel 3: Tablettierte Hartgelatinekapseln mit Oplat-Agonist/Antagonist Extrudaten

**Formulierung:**

| Inhaltsstoff | Menge/Einheit (mg) | Menge/Charge (g) |
|---|---|---|
| Morphin HCl | 12,0 | 120,0 |
| Eudragit NE 30 D | 76,0 | 760,0 |
| Ethylcellulose | 4,5 | 45,0 |
| Stearylalkohol | 27,0 | 270,0 |
| Naloxon HCl | 0,5 | 5,0 |
| Hartgelatinekapsel | √ | √ |
| Gesamt | 120,0 | 1200,0 |

## Patentansprüche

1. Verwendung einer nicht-trennbaren Kombination von Morphin oder dessen physiologisch annehmbaren Salzen und mindestens einem Opiatantagonisten oder dessen physiologisch annehmbaren Salzen mit einer Bioverfügbarkeit von weniger als 5% bei oraler Gabe zur Herstellung eines nur oral zu verabreichenden Arzneimittels zur Substitutionstherapie bei Opiatabhängigen oder Heroinabhängigen, wobei der Opiatantagonist Naloxon ist.

2. Verwendung nach Anspruch 1, **dadurch gekennzeichnet, dass** durch das Arzneimittel auch die opiatspezifischen Nebenwirkungen vermindert oder verhindert werden.

3. Verwendung nach einem der vorangehenden Ansprüche, **dadurch gekennzeichnet, dass** das Morphin retardiert und der mindestens eine Opiatantagonist retardiert ist, das Morphin retardiert und der mindestens eine Opiatantagonist nicht-retardiert ist, das Morphin nicht-retardiert und der mindestens eine Opiatantagonist retardiert ist, das Morphin nicht-retardiert und der mindestens eine Opiatantagonist nicht-retardiert ist.

4. Verwendung nach einem der vorangehenden Ansprüche, **dadurch gekennzeichnet, dass** das Morphin auf einem Polymer adsorbiert und in einer Matrix eingebettet ist oder dass das Morphin in einem Ethylcellulose-Polymer suspendiert ist.

5. Verwendung nach einem der vorangehenden Ansprüche, **dadurch gekennzeichnet, dass** die Substitutionstherapie bei Heroinabhängigen angewendet wird.

6. Verwendung nach einem der vorangehenden Ansprüche, **dadurch gekennzeichnet, dass** es sich um ein einmal oder zweimal täglich einzunehmendes oder zu applizierendes Präparat handelt.

7. Verwendung nach einem der vorangehenden Ansprüche, **dadurch gekennzeichnet, dass** das Morphin in Form des Morphin-Hydrochlorids oder das Morphinsulfat-Pentahydrats eingesetzt wird.

8. Verwendung nach einem der Ansprüche 1 bis 7, **dadurch gekennzeichnet, dass** das Naloxon in Form des Naloxon-Hydrochlorids oder des Naloxon-Hydrochloriddihydrats eingesetzt wird.

9. Verwendung nach einem der vorangehenden Ansprüche, **dadurch gekennzeichnet, dass** das Arzneimittel 100 mg bis 2000 mg retardiertes Morphin enthält.

10. Verwendung nach einem der Ansprüche 1 bis 9, **dadurch gekennzeichnet, dass** das Arzneimittel 0,1 bis 10 mg Naloxon pro 100 mg retardiertes Morphin enthält.

11. Verwendung nach einem der vorangehenden Ansprüche, **dadurch gekennzeichnet, dass** retardiertes Morphin und nicht-retardiertes Naloxon als Granulatmischung vorliegen, wobei die Granulate bevorzugt Korngrößen von 0,1 mm bis 2 mm aufweisen.

12. Verwendung nach vorangehendem Anspruch, **dadurch gekennzeichnet, dass** das Morphin Morphinsulfat-Pentahydrat und das Naloxon Naloxon-Hydrochlorid oder Naloxon-Hydrochlorid-dihydrat ist.

## Claims

1. Use of a non-separable combination of morphine or its physiologically acceptable salts and at least one opiate antagonist or its physiologically acceptable salts having a bioavailability of less than 5% on oral administration, for the manufacture of a medication which is only to be administered orally as substitution therapy for opiate-dependent or heroin-dependent individuals, wherein the opioid antagonist is naloxone.

2. Use according to claim 1, **characterised in that** the medication also reduces or prevents opiate-specific side effects.

3. Use according to one of the preceding claims, **characterised in that** the morphine is retarded and the at least one opiate antagonist is retarded, the morphine is retarded and the at least one opiate antagonist is non-retarded, the morphine is non-retarded and the at least one opiate antagonist is retarded, the morphine is non-retarded and the at least one opiate antagonist is non-retarded.

4. Use according to one of the preceding claims, **characterised in that** the morphine is adsorbed onto a polymer and embedded in a matrix, or **in that** the morphine is suspended in an ethyl cellulose polymer.

5. Use according to one of the preceding claims, **characterised in that** the substitution therapy is used for heroin-dependent individuals.

6. Use according to one of the preceding claims, **characterised in that** the preparation is to be taken or administered once or twice a day.

7. Use according to one of the preceding claims, **characterised in that** the morphine is used in the form of morphine hydrochloride or morphine sulphate pentahydrate.

8. Use according to one of claims 1 to 7, **characterised in that** the naloxone is used in the form of naloxone hydrochloride or naloxone hydrochloride dihydrate.

9. Use according to one of the preceding claims, **characterised in that** the medication contains 100 mg to 2000 mg of retarded morphine.

10. Use according to one of claims 1 to 9, **characterised in that** the medication contains 0.1 mg to 10 mg of naloxone per 100 mg of retarded morphine.

11. Use according to one of the preceding claims, **characterised in that** the retarded morphine and non-retarded naloxone are present as a granular mixture, wherein the granules preferably have grain sizes of 0.1 mm to 2 mm.

12. Use according to the preceding claim, **characterised in that** the morphine is morphine sulphate pentahydrate and the naloxone is naloxone hydrochloride or naloxone hydrochloride dihydrate.

## Revendications

1. Utilisation d'une combinaison non séparable de morphine ou de ses sels physiologiquement acceptables et d'au moins un antagoniste d'opiacé ou de ses sels physiologiquement acceptables avec une biodisponibilité inférieure à 5 % dans l'administration orale, pour la fabrication d'un médicament à administrer uniquement par voie orale pour le traitement de substitution chez des sujets dépendants aux opiacés ou à l'héroïne, où l'antagoniste d'opiacé est la naxolone.

2. Utilisation selon la revendication 1, **caractérisée en ce que**, par le médicament, les effets secondaires spécifiques des opiacés sont également réduits ou empêchés.

3. Utilisation selon l'une des revendications précédentes, **caractérisée en ce que** la morphine est retardée et l'au moins un antagoniste d'opiacé est retardé, la morphine est retardée et l'au moins un antagoniste d'opiacé n'est pas retardé, la morphine n'est pas retardée et l'au moins un antagoniste d'opiacé est retardé, la morphine n'est pas retardée et l'au moins un antagoniste d'opiacé n'est pas retardé.

4. Utilisation selon l'une des revendications précédentes, **caractérisée en ce que** la morphine est adsorbée sur un polymère et est intégrée dans une matrice ou que la morphine est en suspension dans un polymère d'éthylcellulose.

5. Utilisation selon l'une des revendications précédentes, **caractérisée en ce que** le traitement de substitution est employé chez des sujets dépendants à l'héroïne.

6. Utilisation selon l'une des revendications précédentes, **caractérisée en ce qu'**il s'agit d'une préparation à prendre ou à appliquer une ou deux fois par jour.

7. Utilisation selon l'une des revendications précédentes, **caractérisée en ce que** la morphine est employée sous la forme de chlorhydrate de morphine ou de pentahydrate de sulfate de morphine.

8. Utilisation selon l'une des revendications 1 à 7, **caractérisée en ce que** la naloxone est employée sous la forme de chlorhydrate de naxolone ou de dihydrate de chlorhydrate de naxolone.

9. Utilisation selon l'une des revendications précédentes, **caractérisée en ce que** le médicament contient de 100 mg à 2000 mg de morphine retardée.

10. Utilisation selon l'une des revendications 1 à 9, **caractérisée en ce que** le médicament contient de 0,1 à 10 mg de naloxone par 100 mg de morphine retardée.

11. Utilisation selon l'une des revendications précédentes, **caractérisée en ce que** la morphine retardée et la naxolone non retardée se présentent sous la forme d'un mélange de granulés, où les granulés présentent de préférence une taille de grains de 0,1 mm à 2 mm.

12. Utilisation selon la revendication précédente, **caractérisée en ce que** la morphine est le pentahydrate de sulfate de morphine et la naxolone est le chlorhydrate de naxolone ou le dihydrate de chlorhydrate de naxolone.
